# EUROPEAN PATENT SPECIFICATION

(11) **EP 3 400 955 B1**
(45) Date of publication and mention of the grant of the patent: **13.08.2025**
(21) Application number: 17211012.4
(22) Date of filing: 29.12.2017
(51) Int. Cl.: A61K 38/17, A61P 35/00

(54) **USE OF CRYPTONIN FOR TREATMENT OF CANCER**
VERWENDUNG VON CRYPTONIN ZUR BEHANDLUNG VON KREBS
UTILISATION DE CRYPTONINE POUR LE TRAITEMENT DU CANCER

(30) Priority: 29.12.2016 TR 201620086
(43) Date of publication of application: 14.11.2018
(73) Proprietor: Istanbul Universitesi Rektorlugu, 34452 Istanbul (TR)
(72) Inventor: Bas, Serap Sancar, 34134 Istanbul (TR); Bolkent, Sehnaz, 34134 Istanbul (TR)
(74) Representative: Mutlu, Aydin

(56) References cited:
- WO-A2-2013/039861
- KR-A- 20010 077 609
- KR-A- 20150 105 936
- AZMIERA N ET AL: "Antimicrobial peptides isolated from insects and their potential applications", JOURNAL OF ASIA PACIFIC ENTOMOLOGY, KOREAN SOCIETY OF APPLIED ENTOMOLOGY, SUWON, KR, vol. 25, no. 2, 15 February 2022 (2022-02-15), XP087052111, ISSN: 1226-8615, [retrieved on 20220215], DOI: 10.1016/J.ASPEN.2022.101892
- LEEM JAE YOON ET AL: "Isolation and functional analysis of a 24-residue linear alpha-helical antimicrobial peptide from Korean blackish cicada, Cryptotympana dubia (Homoptera).", ARCHIVES OF INSECT BIOCHEMISTRY AND PHYSIOLOGY DEC 2007, vol. 66, no. 4, December 2007 (2007-12-01), pages 204 - 213, XP009505230, ISSN: 0739-4462
- TONK MIRAY ET AL: "Insect antimicrobial peptides: potential tools for the prevention of skin cancer", APPLIED MICROBIOLOGY AND BIOTECHNOLOGY, SPRINGER, DE, vol. 100, no. 17, 15 July 2016 (2016-07-15), pages 7397 - 7405, XP036027529, ISSN: 0175-7598, [retrieved on 20160715], DOI: 10.1007/S00253-016-7718-Y
- HOSKIN ET AL: "Studies on anticancer activities of antimicrobial peptides", BIOCHIMICA ET BIOPHYSICA ACTA (BBA) - BIOMEMBRANES, ELSEVIER, AMSTERDAM, NL, vol. 1778, no. 2, 22 November 2007 (2007-11-22), pages 357 - 375, XP022428150, ISSN: 0005-2736, DOI: 10.1016/J.BBAMEM.2007.11.008
- SEHNAZ BOLKENT: "The effects of some antimicrobial peptides on breast cancer cell lines", TEZ ÖZETLERI ASTRONOMI VE UZAY BILIMLERI ANABILIM DALI, TU, 28 October 2017 (2017-10-28), pages 18 - 20, XP009505243, Retrieved from the Internet <URL:http://muhaz.org/tez-ozetleri-astronomi-ve-uzay-bilimleri-anabilim-dal-v2.html?page=3>

## Description

### Technical Field

The present invention relates to use of a peptide for treatment of cancer. The present invention particularly relates to use of a peptide having SEQ ID NO: 1, particularly cryptonin, for treatment of cancer, which leads to necrotic cell death by degrading membrane of the cancer cell.

### Background of the Invention

Cryptonin is an antimicrobial peptide having α-helix structure defined in the hemolymph of cicada (Cryptotympana dubia) and a net charge of +8. It has 24 amino acids in length. Its antimicrobial activity against Gram-negative and Gram-positive bacteria and fungi is known. Its amino acid sequence is Gly-Leu-Leu-Asn-Gly-Leu-Ala-Leu-Arg-Leu-Gly-Lys-Arg-Ala-Leu-Lys-Lys-Ile-Ile-Lys-Arg-Leu-Cys-Arg.

Cancer is a disease that is highly variable and occurs by heterozygous genetic and epigenetic changes. The onset and development of a cancer is characterized by various mutations, chromosomal defects and increased gene expressions. Increased transcript levels in cancerous genomes are associated with the number of gene copies increased with the proliferation of oncogenes, and the inactivation of tumor suppressor genes.

Breast cancer is also assessed in the same context Breast cancers consist of subgroups whose response to treatment is variable due to the different molecular characteristics of the individuals. Therefore, it is of particular interest in the "personal treatment" researches. Breast cancer is the most common cancer in women and the second most common cancer overall. There are histologically two types, invasive and non-invasive. Non-invasive cancers are ductal carcinoma in situ and lobular carcinoma in situ, whereas invasive cancers are invasive ductal carcinoma and invasive lobular carcinoma, tubular carcinoma, invasive cribriform carcinoma, medullar carcinoma, mucinous carcinoma, neuroendocrine carcinoma, invasive papillary carcinoma, invasive micropapillary carcinoma, apocrine carcinoma, metaplastic carcinoma, lipid-rich carcinoma, secretory (juvenile) carcinoma, oncocytic carcinoma, and adenoid cystic carcinoma.

Breast cancer is the most common type of cancer among all women with more than a million new cases every year in the world. It is more common in the developed western societies with an incidence of more than 80 in 100,000 people, whereas in the recent years, the incidence rate has been increased by 5% in the developing countries such as Asia, Africa and part of Europe, which have previously lower incidence rates. The American Cancer Society estimates that there will be 226,870 new cases of invasive breast cancer in the United States in 2012, the number of cases which will be detected in very early stage would be 63,300 and 39,510 people would be deceased (www.cancer.org, 2012).

An important clinical problem in breast cancer is the progression of breast tumors that initially respond to both hormonal and chemotherapeutic approaches to a more aggressive stage by responding weakly to agents in both categories. For this reason, the necessity of antineoplastic agents with different mechanism of action is inevitable.

Today, many cancer drugs used in chemotherapy are divided into different groups such as alkylating agents, antimetabolites, antitumor antibiotics, nitrosoureas, plant alkaloids and hormones according to their specific properties and effects on cancer cells. The drug concentrations required to control tumor growth also affect normal cells and cause many side effects. Many of these agents reach the cytosol and interact with their intracellular targets. However, the resistance mechanism developed by tumor cells carries these agents out of the cell before they interact with their intracellular targets. This lowers the therapeutic value of anticancer drugs used today. It is also known that some therapeutic drugs such as tamoxifen cause secondary malignancy. On the other hand, tumor cells develop a different resistance mechanism by changing the pathways of signal transduction against drugs targeting growth factors on their cell surface. Therefore, there is an urgent need to develop new anticancer drugs that act selectively on cancer cells, independent of the cell proliferation capacity, the specific signal transduction pathways, and the presence of proteins that provide resistance against the drug by pumping it out.

Due to the disadvantages mentioned above, a novelty is needed in the technical field related to the treatment of cancer, especially of breast cancer.

### Summary of the Invention

The present invention relates to the use of a peptide having SEQ ID NO:1, for use in treatment or prophylaxis of cancer. Said peptide is preferably cryptonin.

The cancer described herein may be selected from breast cancer, prostate cancer, skin cancer, gastric cancer, liver cancer, kidney cancer, lung cancer, colon cancer, ovarian cancer, testis cancer, bone cancer, ureter cancer, cervical cancer, endocrine gland cancers, vaginal cancer, endometrial cancer, duodenal cancer, head and neck cancer, Hodgkin's disease, vulva cancer and esophageal cancer. However, more preferably treatment and prophylaxis of breast cancer is targeted.

Said breast cancer may be a disease selected from ductal carcinoma in situ and lobular carcinoma in situ, invasive ductal carcinoma and invasive lobular carcinoma, tubular carcinoma, invasive cribriform carcinoma, medullar carcinoma, mucinous carcinoma, neuroendocrine carcinoma, invasive papillary carcinoma, invasive micropapillary carcinoma, apocrine carcinoma, metaplastic carcinoma, lipid-rich carcinoma, secretory (juvenile) carcinoma, oncocytic carcinoma, and adenoid cystic carcinoma.

The peptide of the invention may contain 24 amino acids, and has preferably a linear structure. The amino acids in the peptide are preferably in D-form and have a net charge of +8.

The peptide of the invention preferably treats breast cancer by inducing necrotic cell death.

In a further aspect, the invention relates to a combination comprising said peptide and at least one chemotherapeutic agent, or a pharmaceutical composition comprising at least one excipient. Said chemotherapeutic agent may be selected from paclitaxel, docetaxel, vinorelbine, vincristine, vinblastine, doxorubicin, epirubicin, bleomycin, cisplatin, pentostatin, gemcitabine, mitomycin, etoposide, teniposide, topotecan, hydroxyurea, idarubicin, lomustine, thalidomide, 5-azacytidine, carboplatin, bortezomib, melphalan, 6-mercaptopurine, carmustine, oxaliplatin, daunorubicin, chlorambucil, irinotecan, semustine, cytarabine and busulfan. Said excipients may be selected from the group consisting of diluents, binders, disintegrants, glidants, lubricants, solvents, pH adjusters, gelling agents and plasticizers.

Pharmaceutical compositions of the invention may be in solid or liquid or topical liquid or in gel form.

### Brief Description of the Figures

Figure 1 shows the effect of cryptonin on cell viability in MCF-7 and MDA-MB 231 cells.
Figure 2 shows the effect of cryptonin on the release of lactate dehydrogenase enzyme in MCF-7 cells.
Figure 3 shows the effect of cryptonin on the release of lactate dehydrogenase enzyme in MDA-MB 231 cells.
Figure 4 shows the effect of cryptonin on caspase-3 activity in MCF-7 cells.
Figure 5 shows the effect of cryptonin on caspase-3 activity in MDA-MB 231 cells.
Figure 6: A) shows the dose-dependent number of ethidium bromide stained necrotic cell nuclei in MCF-7 cells. B) shows that cryptonin increases the number of necrotic cells in a dose-dependent manner, while reducing the number of viable cells in MCF-7 cells.
Figure 7 shows the binding of biotinylated cryptonin to heparan sulphate and chondroitin sulphate.

### Detailed Description of the Invention

In this detailed description, the peptide of the invention and use thereof for the cancer treatment is described only for better understanding of the subject matter, without any limitation.

In said treatment, the effects of cryptonin have been investigated in vitro in cancerous cells, in particular breast cancer cells.

Cryptonin is an antimicrobial peptide having α-helix structure defined in the hemolymph of cicada (Cryptotympana dubia) with a net charge of +8. It has 24 amino acids in length. Its antimicrobial activity against Gram-negative and Gram-positive bacteria and Fungi is known. Its amino acid sequence is Gly-Leu-Leu-Asn-Gly-Leu-Ala-Leu-Arg-Leu-Gly-Lys-Arg-Ala-Leu-Lys-Lys-Ile-Ile-Lys-Arg-Leu-Cys-Arg.

SEQ ID NO: 1 is referred as GLLNGLALRLGKRALKKIIKRLCR. Each single letter represents an amino acid.

Possible electrostatic interactions between membrane and peptide are planned to be realized by cationic and amphipathic character. The anticancer activity of this antimicrobial peptide has been investigated on MCF-7 and MDA-MB 231 cells, two different breast cancer lines that are used frequently in *in vitro* breast cancer studies. With this study;
1. Anticancer activities of cryptonin peptide on the breast cancer cells have been revealed.
2. Its effect on mouse fibroblast cell line (3T3) was investigated to determine whether its anticancer activities were selectively present in cancer cells.
3. It has been determined that the cell death to be induced by antimicrobial peptides is related to which type of cellular death.
4. Anticancer peptide-membrane interactions are revealed in toxicities that occur in cancer cells.

The intensive use of traditional chemotherapeutic agents leads to the emergence of resistant tumor cells. Therefore, one of the biggest problems that lead to failure in cancer therapy is resistance against the drug. Resistance to anticancer drugs can be addressed in two ways. The first one is intrinsic resistance, which is due to individual differences in the patients and differences in somatic cell genetics in the tumor. The second one is more commonly observed acquired resistance. The most important reason for the acquired resistance to many cancer drugs is the expression of one or more energy-dependent carriers such as membrane-bound efflux pump, P-glycoprotein. These carrier proteins inactivate Adriamycin and vinca alkaloids. In addition, these cells are also cross-resistant to antitumor drugs to which they have not previously been exposed (Wang et al., 2009). On the other hand, even if the drugs are taken into the cell, the efficacy of the drug may be reduced by the loss of specific enzyme activity related to the metabolic activation of the drug, or by the increase of the activities of the enzymes that convert the drug into inactive metabolites. In addition, cancer cells can modify intracellular targets of the drug by increasing the expression levels, or acquiring mutations that change their function. Drug resistance may develop due to the defects in apoptosis pathways of the tumor cells, or as a result of repair of macromolecule damages caused by a drug, by the tumor cells (Kudoh et al., 2000; Kruh, 2003). Thus, a great deal of effort is being made to search for effective agents against resistant cells, and identification, and development of novel agents having a mechanism of action that selectively target cancer cells is of great importance.

It is stated that the cationic antimicrobial peptides bind rapidly to many cancer cells, including the drug-resistant cancer cells, and kill these cells without harming the vital organs. Therefore, these peptides with an anti-cancer activity have the potential to be superior to conventional chemotherapeutic agents (Johnstone et al., 2000; Kim et al., 2003; Donelly, 2004; Hansel et al., 2007).

The majority of the researches for breast cancer are carried out in breast cancer cell lines as model systems. These lines are usually classified according to whether they contain an estrogen receptor (ER) or not. In this study, an ER(+) or hormone dependent cell line MCF-7, and an ER(-) hormone independent cell line MDA-MB 231 cells were used.

MCF-7 is the most commonly studied and best characterized cell line among other breast cancer cell lines. This cell line was first isolated from a 69-year-old patient with malignant adenocarcinoma by pleural effusion in 1970. This cellular model is used in most studies performed to understand the mechanisms of action of estrogen and antiestrogens, and their role in regulating the proliferation of hormone dependent cancer cells. MCF-7 cells may form tumors when 17-p estradiol is administered to nude mice (without hair and without thymus) (Shafie and Liotta, 1980). Another cell line used in this study is MDA-MB 231. This cell line is estrogen-negative and carries p53 mutation, and also is more invasive than MCF-7 cells, and can produce tumors when injected into fat pad of nude mice, or metastasize when injected into the tail vein (Mukhopadhyay et al., 1999).

The effect of cryptonin peptide on viability of 3T3 mouse fibroblast cells representing breast cancer cells and normal cells was investigated by 3-(4,5-dimethylthiazol-2-yl)-2,5-diphenyltetrazolium bromide (MTT) test. Its possible lytic effect on the cancer cell lines was determined by measuring the release of lactate dehydrogenase. As certain peptides, the anticancer activity of which have been investigated, induce apoptosis in the cancer cell lines, possible apoptosis inducing activities of these peptides have been revealed by measurement of DNA fragmentation and colorimetric measurement of caspase 3 activity. Type of cell death occurred with antimicrobial peptides was determined morphologically in a fluorescence microscope by ethidium bromide/acridine orange double staining technique. Possible membrane damage caused by the direct action of antimicrobial peptides in cancer cell lines has been understood by morphological observation of cells in a scanning electron microscope. Affinity of antimicrobial peptide, the anticancer activity of which has been investigated, for proteoglycans such as heparin sulfate and chondroitin sulfate which have been shown to be more expressed on cancer cell surfaces was determined by the solid phase heparin sulfate and chondroitin sulfate binding method using biotin- conjugated antimicrobial peptide. Finally, the investigation of the antimicrobial peptide affinity for cancer cell membranes and fibroblast cell membranes has also been demonstrated by the peptide binding assay method using biotin-conjugated peptides.

In this study, cultures of mouse 3T3 fibroblast cells representing normal cells and cultures of MCF-7 and MDA-MB 231 cell lines, two different breast cancer cell lines, were performed. Cells were grown in flasks containing 1:1 mixture of Dulbecco's modified Eagle's medium (DMEM) supplemented with 10% fetal bovine serum and antibiotic (100 U/ml of penicillin G, 100 µg/ml of streptomycin) and F12 medium, under a pressure of 1 atmosphere at 37°C in a 5% CO2 incubator. When the grown cells covered the flask, the media in the flask was decanted and the cells were washed with sterile calcium-magnesium free PBS. Cells were removed with 2.5% sterile trypsin-EDTA solution and trypsin activity was inhibited by media addition. Cells were then collected by centrifugation at 1500xg for 3 minutes and passage was performed in new flasks, or the appropriate number of cells were counted on the Thoma hemocytometer and plated on plates.

Cells were frozen for long-term storage. For this procedure, the cells grown in the flask were removed with trypsin and the total number of cells on the Thoma slide was calculated. After counting the cells, the cells were collected by centrifugation at 1500xg for 3 minutes. At this time, the freezing medium was prepared. Cold fetal bovine serum (FBS) containing 10% dimethyl sulfoxide (DMSO) used as a cryoprotectant agent was used as freezing medium. The cell pellet was suspended in the freezing medium at a concentration of approximately 1.5-2 million cells/ml and the cell suspension was transferred to 2 ml cryotubes. The cryotubes are frozen at -87°C for long term storage after standing at -20°C overnight. When the frozen cells had to be opened, the cryotubes were rapidly brought to 37°C and the cell suspension in the freezing medium was quickly transferred to a centrifuge tube containing 5 ml of medium, and the cells were centrifuged at 1500xg for 3 minutes. The resulting cell pellet was washed again in 5 ml of medium to remove the DMSO and centrifuged again. The second cell pellet was suspended in a small volume of medium and transferred to flasks of 25 or 75 cm². Cell viability was controlled with 4% trypan blue prepared in PBS at each cell opening.

### Dissolution and Reconstitution of Peptide

The biotin-free or biotinylated peptide used in this study was obtained from Invitrogen and Genecust in at least 95% pure and lyophilized form. In the study, Cryptonin was dissolved according to its charge and hydrophobicity status.

Cryptonin was dissolved at a density of 1 mg/ml in PBS (pH 7.4). For sterilization, the solution was filtered through a membrane filter with a porosity of 0.22 µm, aliquoted and brought to -20°C.

### Cell Viability Test

Thiazolyl blue tetrazolium bromide (MTT) test was performed to investigate the effect of cryptonin on the viability of MCF-7, MDA-MB 231 and 3T3 fibroblast cell lines (Mosmann, 1983). Yellow MTT is reduced to purple formazan crystals by the activity of mitochondrial dehydrogenases in viable cells. The intensity of the resulting purple color is directly proportional to the number of viable cells. MTT test was performed in 96-well plates. To this end, the cells grown in the flasks were removed by trypsin-EDTA, then collected by centrifugation and the cell pellet was suspended in the medium such that 5000 cells would be present per 100 µl. 5000 cells were plated in each well of the plate. In the following day, the medium in the wells was removed, fresh medium was added to the control group, and a medium containing anti-microbial peptide was added to the experimental groups at 8 different concentrations. This medium contained 10% serum.

Cells were exposed to different concentrations of antimicrobial peptide for 24 hours. At the end of this period, 30 µl of a solution of 3-(4,5-dimethylthiazol-2-yl)-2,5-diphenyltetrazolium bromide (MTT) was added to each well in the plate. MTT solution was prepared in PBS (pH 7.4) at a density of 5 mg/ml and sterilized by passing through a membrane filter. After MTT-containing medium was incubated in the dark for 4 hours at 37°C in a carbon dioxide incubator, medium with MTT was drawn from all wells. Upon reduction of MTT, the formazan crystals formed in the wells were dissolved by the addition of 100 µl of DMSO to all wells and the plates were shaken on an orbital shaker for 5 minutes at 150 rpm. The optical density was measured at a test wavelength of 570 nm and a reference wavelength of 630 nm. In this test, 4 wells were run in each plate for each of 8 different concentrations of cryptonin, and each experiment was repeated 3 times. The mean absorbance values obtained from the control group were accepted as 100% cell viability and the values obtained from the wells treated with antimicrobial peptide were assessed versus control group.

### Measurement of Lactate Dehydrogenase Release

The lactate dehydrogenase (LDH) release assay is based on the measurement of a cytoplasmic enzyme, lactate dehydrogenase, activity released from damaged cells (Lobner, 2000) and is used for the assessment of cellular cytotoxicity. The cytotoxic effect of cryptonin on MCF-7 and MDA-MB 231 breast cancer cell lines was determined by measuring the activity of lactate dehydrogenase (LDH) enzyme released from damaged cells and a Cytotoxicity Detection Kit, LDH (Roche Molecular Diagnostics, Germany) was used for this purpose.

In LDH test, a negative control, a control group for spontaneous LDH release from the cells, and a positive control group for maximum LDH release were studied, as well as experimental groups to which peptide was administered. For the effect of cryptonin on cell lysis, doses close to the dose that reduces the cell viability by 50% according to MTT results were studied. For this purpose, doses of 10, 25, 50 and 100 µg/ml were administered for MCF-7 cells and doses of 7.5, 10, 25 and 50 µg/ml were administered for MDA-MB 231 cells, for 24 hours. Cells were grown in the flasks of 75 cm, then removed with trypsin-EDTA and centrifuged. Cells were suspended in medium at 5000 cells per 100 µl, and 100 µl of cell suspension was transferred to 96-well plates. All control groups and experimental groups were studied with 3 wells and the experiment was repeated 3 times. After a 24-hour incubation, the media of the control groups were refreshed. The medium of the experimental groups was replaced with a medium containing different concentrations of peptide. The lactate dehydrogenase enzyme activity released from the cells after the cells were incubated with cryptonin for 24 hours was determined following the manufacturer's instructions. 5 µl of lysis solution (2% Triton-X-100) was added to the wells of the positive control group and the plate was incubated for 15 minutes at 37°C. To measure LDH activity, 100 µl of the freshly prepared reaction mixture containing catalyst (Diaphorase/NAD⁺ mixture) and dye solution (iodotetrazolium chloride (INT) and sodium lactate) was added to each well. After 30 minutes of incubation in the dark at room temperature, the reaction was stopped by the addition of 50 µL of stop solution (1N HCl) to each well. The plates were shaken for 10 seconds on an orbital shaker and the spectrophotometric absorbance was measured at a test wavelength of 490 nm and a reference wavelength of 690 nm. The results were determined as % cytotoxicity. The mean absorbance values of the negative control were subtracted from the control and experimental groups, the positive control group was considered to be 100% cytotoxic and the cytotoxicities were calculated by comparing the control and experimental groups with positive control group using the following formula: % Cytotoxicity: Control/Experimental - Negative control x 100 (Xiong et al., 2007) Positive control - Negative control

### Measurement of Caspase-3 Activity

It was aimed to measure caspase-3 activity in MCF-7 and MDA-MB 231 cell lines to determine whether the reduction in cell viability previously determined by incubation of cryptonin is caspase dependent. Cryptonin concentrations of 10, 25, 50 and 100 µg/ml were incubated with breast cancer cell lines. For this purpose, firstly the cells were plated with one million cells being present in each well of a 6-well plate. After 24 hours, a peptide solution at three different concentrations of 50, 100 and 200 µg/ml was prepared from a stock antimicrobial peptide solution (1 mg / ml) in serum-containing DMEM-F12, and applied to the experimental groups. The medium of the control group was replaced with a fresh medium without antimicrobial peptide. Caspase-3 enzyme activity was measured with the ApoTarget Caspase-3 Protease Assay kit (Invitrogen). After 24-hour incubation, the cells were removed with trypsin-EDTA and transferred to the falcon tubes. After counting the cells in the Thoma slide, they were centrifuged at 1500 g for 3 minutes and the cell pellet was lysed on ice for 10 minutes in 50 µl of cold cell lysis buffer. After centrifugation at 10,000 g for one minute at +4°C, the cell lysate was collected as supernatant and stored at -20°C in order to measure the caspase activity.

Prior to measuring caspase activity, the protein concentration in the cell lysate was determined by the Bradford method and bovine serum albumin (BSA) was used as standard. Five different standard solutions of 8, 10, 20, 40 and 80 µg/ml were prepared from the stock solution at a concentration of 1.46 mg/ml. 160 µL of standard BSA solutions and samples diluted with distilled water in a ratio of 1:50 were added to three wells each of a 96-well plate. Distilled water was used as the blank. 40 µl of concentrated Bradford solution was added to all wells and pipetted. After 5 minutes, absorbance at a wavelength of 595 nm was measured on a BioTek Microplate reader. A standard graphic was plotted using the absorbance values obtained against the concentrations of standard BSA solutions. With this graphic, the protein concentration of the samples was calculated. Cell lysis was diluted such that 200 µg of protein was present per 50 µl of the cell lysis buffer and caspase-3 activity was measured in 50 µl of cell lysate. 50 µl of 2X reaction buffer containing 10 mM dithiothreitol was added to 50 µl of cell lysate and to this mixture was then added 5 µl of 4 mM DEVD-pNA and the samples were incubated for 2 hours at 37°C in the dark. DEVD-pNA was not added to some samples used as negative control. The spectrophotometric absorbance of the samples was measured at 405 nm on a BioTek microplate reader. Caspase-3 activity was determined as fold increase by comparing with the activity in the control group.

### DNA Fragmentation Analysis

Agarose gel electrophoresis was performed for analysis of DNA fragments that may occur in cell death caused by cryptonin. Doses of 25, 50, 100 and 200 µg/ml of cryptonin were applied on the cells. MCF-7 and MDA-MB 231 breast cancer cell lines were plated in 6-well plates such that one million cells would be present in each well. After 24-hour incubation, a fresh medium containing antimicrobial peptides at different concentrations was applied to the cells. After 24 hours, the floating cells were collected. In order to remove the cells, trypsin-EDTA was applied to the cells after washing with calcium-magnesium-free PBS. Adherent cells and floating cells were collected in the same centrifuge tube and then centrifuged at 4100 rpm for 3 minutes. The cell pellet obtained after centrifugation was suspended in 200 µl of PBS. The DNAseasy Blood & Tissue kit (Qiagen) was used for DNA isolation from the cells. 20 µl of Proteinase K and 4 µl of RNase A were added to the cell suspension and the mixture was homogenized by vortexing and allowed to stand at room temperature for 2 minutes. Then, 200 µL of buffer AL was added to the tubes and homogenization was achieved by mixing the tubes again by vortexing. After incubation at 56°C for 10 minutes with shaking, 200 µl of ethanol (96%) was added to all the samples and the mixture was homogenized by vortexing. This mixture was transferred to a DNeasy Mini spin column placed in a collecting tube of 2 ml. The spin column was centrifuged at 8000 g at room temperature for 1 minute. After centrifugation, the collecting tube and the liquid therein were disposed. The DNeasy mini spin column was placed in a new 2 ml collecting tube and 500 µl of buffer AW1 was added. Centrifugation was again performed at 8000 g at room temperature for 1 minute. The collecting tube and the liquid therein were disposed. The DNeasy mini spin column was placed in a new 2 ml collecting tube and 500 µl of buffer AW2 was added. The spin column was centrifuged at 20,000 g at room temperature for 3 minutes. The collecting tube and the liquid therein were disposed. The DNeasy mini spin column was transferred to a clean eppendorf tube of 1.5 ml. 50 µL of buffer AE was added onto the DNeasy membrane in the spin column. The spin column was incubated at room temperature for 1 min and then centrifuged at 8000 g at room temperature for 1 min to elute the DNA. The DNA concentration in the elution buffer was measured using the Nanodrop 2000c (Thermo Scientific) instrument. Ratio of DNA to RNA of 1,75 and above was considered to be pure.

1% gels were prepared for the visualization of the DNA isolated from the cells. 0.5 g of agarose was dissolved in 50 ml of 1X Tris-Acetic-EDTA (TAE) buffer using a microwave oven and 2.5 µl of ethidium bromide (10 mg/ml) (Sigma) solution was added. After the agarose solution was shaken, it was poured into a gel cassette and allowed to cool for 20-30 minutes. Once the gel in the cassette was placed in the device, the tank was filled with TAE buffer, and the DNA ladder and DNA samples were loaded into the wells by mixing with the DNA loading stain. Gel was run at 120 volts for 30 minutes. The DNA in the agarose gel was analyzed and visualized with the Gel Imaging System (Kodak Molecular Imaging GL 1500).

### Morphological Determination of the Type of Cell Death by Acridine Orange/ Ethidium Bromide

Cells were stained with acridine orange/ethidium bromide double staining method to morphologically analyze whether cryptonin-induced cell death occurs by a necrotic pathway or by an apoptotic pathway. For this purpose, breast cancer cells were plated in a 24-well plate, with 10,000 cells being present in each well and incubated for 24 hours at the above-mentioned different concentrations of antimicrobial peptides. After incubation, a mixture of acridine orange (Sigma) (100 µg/ml) /ethidium bromide stain (Sigma) (100 µg/ml) prepared in PBS was applied to the cells and the cells were visualized using a FITC/CY3 filter on a fluorescent microscope (Nikon DS5MCU2). 200 cells from each group were counted according to the staining pattern. Cells were grouped as viable, apoptotic and necrotic according to the staining pattern of their nuclei (Spector et al., 1998). Viable cells were distinguished as having green chromatin nuclei in an organized structure, apoptotic cells were distinguished as having green or orange fragmented nuclei, and necrotic cells were distinguished as normal colored but having orange nuclei like normal cells (Ribble et al., 2005).

### Determination of Membrane Damages of Peptide-Treated Cells by Scanning Electron Microscope

MCF-7 and MDA-MB 231 and 3T3 fibroblast cells were plated into sterile round slides with a diameter of 12 mm in the wells in 24-well plate such that each well contained 50,000 cells. The following day, the medium was replaced with a medium containing cryptonin at a concentration of 100 µg/ml. After 24-hour incubation, the cells were washed 2 times with 0.1 M sodium cacodylate buffer (pH 7.2) and fixed with 2.5% glutaraldehyde prepared in 0.1 M sodium cacodylate buffer for 2 hours. After the first fixation, the cells were washed again with 0.1 M sodium cacodylate buffer and fixed with 1% osmium tetroxide prepared in 0.2 M sodium cacodylate buffer for 30 minutes for the second time. After the cells were washed with 0.2 M sodium cacodylate buffer, they were taken in an increasing series of ethyl alcohol. Dehydration was performed by incubation 3 times with 30% alcohol for 5 minutes, 3 times with 50% alcohol for 5 minutes, 2 times with 70% alcohol for 10 minutes, 2 times with 85% alcohol for 15 minutes, 2 times with 95% alcohol for 15 minutes and 3 times with 100% alcohol for 10 minutes. After the slides were air-dried, they were coated with golden by BioRad SC 502 coating device. The cells were examined with Jeol Neoscope JCM-500 scanning electron microscope and their photographs were taken (Vitale et al., 1998; Hilchie et al., 2011).

### Measurement of Solid Phase Heparan Sulphate and Chondroitin Sulphate Binding

The binding capacity of cryptonin to glycoconjugates such as heparan sulphate and chondroitin sulphate, which play a role for the cancer cell membranes having negative charge, was measured by a method, a modification of the modified enzyme-linked immunosorbent assay (ELISA), by Silvestri and Sundqvist (2001). For immobilization of the polysaccharides, 96-well plates were coated overnight with 100 µl of heparan sulfate and chondroitin sulfate prepared in PBS (pH 7.4) at a concentration of 10 µg/ml, at room temperature overnight. The following day, the wells were washed twice with PBS (PBST) containing 0.05% Tween 20. To the wells were added a medium with different concentrations of biotin-conjugated peptide in 100 µl, and incubated for 2 hours at 37°C. After incubation, the wells were washed again with PBST. Avidin-conjugated horseradish peroxidase (HRP) was diluted with PBST (Elisa Buffer) containing 0.5% bovine serum albumin (BSA) at a ratio of 1: 250, and transferred to be 100 µl per well. After incubation for 2 hours at room temperature, the wells were washed again with PBST and 100 µl of 3,3', 5,5-Tetramethylbenzidine (TMB) substrate was transferred to the wells. After incubation for 10 minutes at room temperature, the color reaction was stopped by the addition of 50 µl of 1 N hydrochloric acid (HCl) to the wells and absorbance was measured with a Biotek microplate reader at 450 nm.

### Measurement of Binding Affinity of the Peptide for MDA-MB 231 and 3T3 Fibroblast Cells

After the breast cancer cell lines and 3T3 fibroblast cells were grown in the flasks of 75 cm², they were plated onto sterile slides in 24-well plates, with 50,000 cells being present in each well. After 24 hours of cell adhesion, a medium containing biotin-conjugated peptide was applied to the cells at concentrations of 100 and 200 µg/ml for which a significant affinity for the cells was detected. For control purposes, some wells were treated with an antimicrobial peptide-free medium. After 1-hour incubation with antimicrobial peptide, cells were fixed with 4% paraformaldehyde fixative for 20 minutes. The fixative was then removed and the cells washed with PBS (pH 7.4) 3 times for 10 minutes. Cells were incubated with Texas Red-conjugated streptavidin (Invitrogen) at a concentration of 5 µg/ml prepared in PBS for 45 minutes. After washing with PBS 3 times for 10 minutes, the coverslips were mounted onto the slides with fluorescence mounting medium (Invitrogen) and photographed on a fluorescent microscope (Nikon DS5MCU2) using a Cy3 filter. The average fluorescence intensity in each cell was determined using NIS-Elements (Nikon) software.

### Statistical Analysis

The results were evaluated using the GraphPad Prism 5.0 computer program. The results were given as mean ± SD (standard deviation) and statistically analyzed by student-t test for paired comparisons. For multiple comparisons, between-group homogeneity was statistically compared by One-Way ANOVA test, and Bonferroni's multiple comparison test was used to determine whether there was a significant difference between the averages of the groups. Microsoft Excel 2007 program was used for plotting the graphs.

### Results for Cell Viability Test of Cryptonin

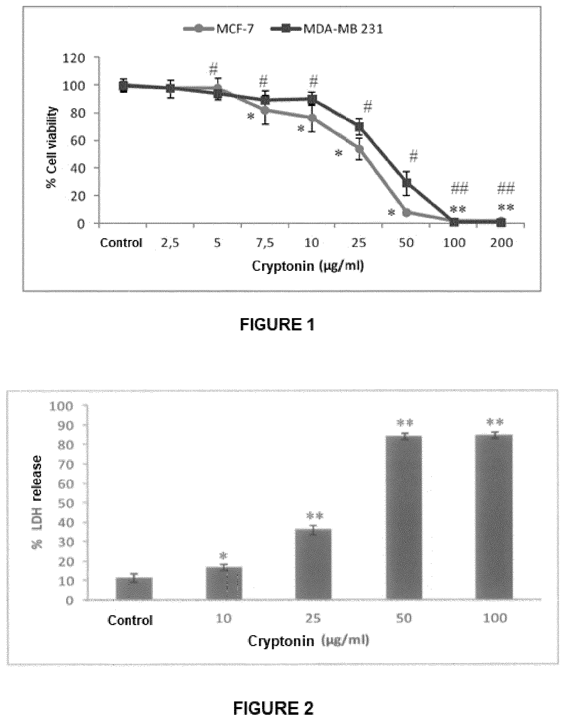

Figure 1 shows the effect of cryptonin on the cell viability in MCF-7 and MDA-MB 231 cells. *A significant decrease for MCF-7 was observed compared to the control group (*p<0.05, **p<0.001). Values for MCF-7 are: Control: 100:1:4.3, 2.5 µg*/*ml: 97.6±6.6, 5 µg*/*ml: 98.24±7.4, 7.5 µg*/*ml: 82.2±10.3, 10 µg*/*ml: 76.57±9.9, 25 ug*/*ml: 53.95±7.5, 50 µg*/*ml: 7.82±1.8, 100 ug*/*ml: 1.16±0.5, 200 µg*/*ml: 1.81±0.9. A significant difference for MDA-MB 231 was observed compared to the control group (# p<0.05; ## p<0.001). Values for MDA-MB 231 are: Control: 100±3.5, 2.5 µg*/*ml: 97.28±2.2, 5 µg*/*ml: 94.34±4.5, 7.5 µg*/*ml: 89.34±6.6, 10 µg*/*ml: 90.38±4.8, 25 µg*/*ml: 70.25±5.8, 50 ug*/*ml: 29.31±8.7, 100 µg*/*ml: 0.99±0.06, 200 µg*/*ml: 0.65±0.1.*

According to the graph of Figure 1, cryptonin decreased the cell viability in MCF-7 and MDA-MB 231 cell lines in a dose-dependent manner. In MCF-7 cells, the cell viability was significantly reduced at doses of 25, 50, 100 and 200 µg/ml (p<0,001) as compared to doses of 7.5 and 10 µg/ml (p <0.05). In MDA-MB 231 cells, more reduction in the cell viability was observed at doses of 25, 50, 100 and 200 µg/ml (p<0,001) as compared to doses of 5, 7.5 and 10 µg/ml (p<0,05). Similarly, cryptonin reduced the number of viable cells in 3T3 fibroblast cells with being less at a dose of 10 µg/ml, but more at doses of 25, 50, 100 and 200 µg/ml.

### Effect of Cryptonin on the Release of Lactate Dehydrogenase

Figure 2 shows the effect of cryptonin on the release of lactate dehydrogenase enzyme in MCF-7 cells. *A significant increase in LDH release was observed as compared to the control group (*p<0.05, **p<0.001). Control: 11.54±2.2, 10 ug*/*ml: 16.9:1:1.6, 25 µg*/*ml: 36.03±2.1, 50 ug*/*ml: 84.1±1.7, 100 µg*/*ml: 84.66±1.6.*

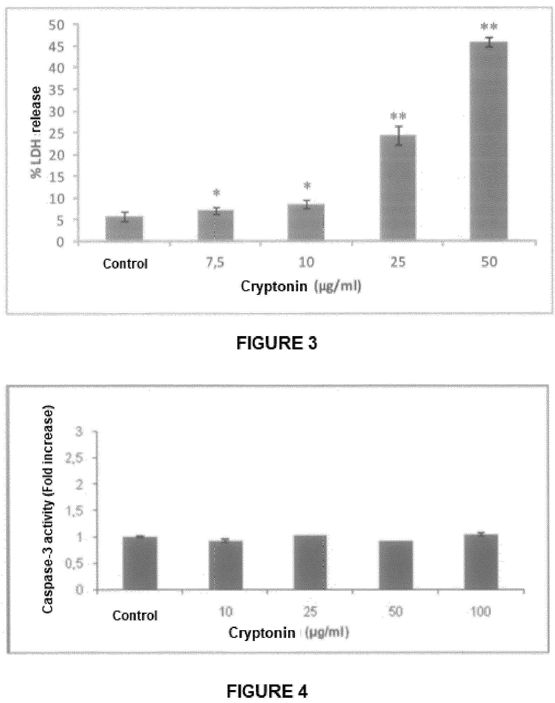

Figure 3 shows the effect of cryptonin on the release of lactate dehydrogenase enzyme in MDA-MB 231 cells. *A significant increase in LDH release was observed as compared to the control group (*p<0.05, **p<0.001). Control: 5.7±1, 7.5 µg*/*ml: 7.09±0.7, 10 µg*/*ml: 8.46±0.9, 25 µg*/*ml: 24.24±2.1, 50 µg*/*ml: 45.90±1.*

Cryptonin has increased the release of lactate dehydrogenase enzyme in both breast cancer cell lines in a dose-dependent manner. When LDH release of both cell lines was compared, it has been observed that MCF-7 cells release more enzyme at doses of 10, 25 and 50 µg/ml as compared to MDA-MB 231 cells.

### Effect of Cryptonin on Caspase-3 Activity

*Figure 4* *shows the effect of cryptonin on caspase-3 activity in MCF-7 cells. No significant difference was observed in caspase-3 activity as compared to the control groups (p>0.05). Control: 1±0.01, 10 µg*/*ml: 0.93±0.03, 25 µg*/*ml: 1.03±0.50, 50 µg*/*ml: 0.92±0, 100 µg*/*ml: 1.05±0.03.*

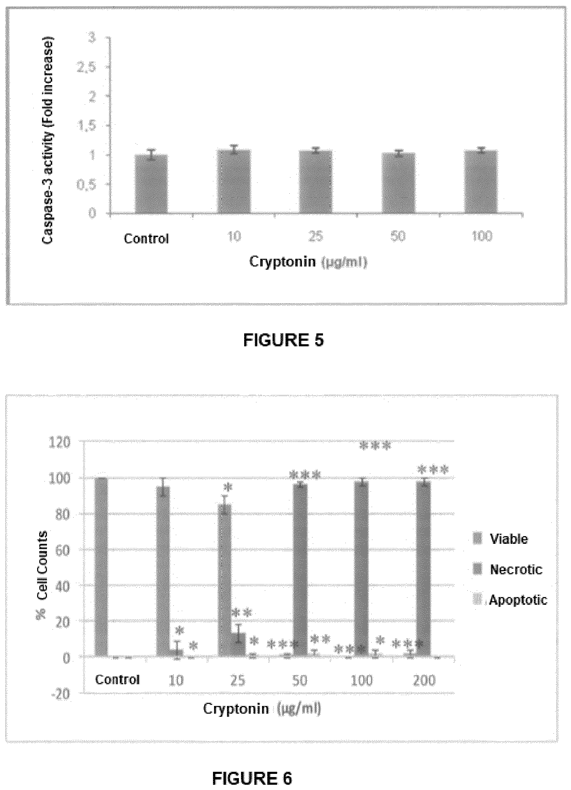

Figure 5 shows the effect of cryptonin on caspase-3 activity in MDA-MB 231 cells. *No significant difference was observed in caspase-3 activity as compared to the control groups (p>0.05). Control: 1±0.08, 10 µg*/*ml: 1.08±0.07, 25 µg*/*ml: 1.07±0.04, 50 µg*/*ml: 1.02±0.05, 100 µg*/*ml: 1.06±0.04.*

No significant difference was observed in caspase-3 activity in MCF-7 and MDA-MB 231 cells for cryptonin-induced cell death as compared to the control group (p>0.005).

### Effect of Cryptonin on DNA Fragmentation

No fragmentation was observed in the DNA isolated from the cells after 24-hour incubation with different doses of cryptonin.

### Results for Morphological Determination of the Type of Cell Death by Acridine Orange/Ethidium Bromide

Cryptonin has reduced the number of viable cells and increased the number of necrotic cells in a dose-dependent manner in both cell lines. A significant decrease in the number of viable cells and a significant increase in the number of necrotic cells were observed during the counts performed by nucleus staining and morphology. Apoptotic cells were slightly increased in number in MCF-7 cells at concentrations of 10, 25, 50 and 100 µg/ml. No significant increase in MDA-MB 231 cells was observed.

**Table 1 Mean percentage values of the viable, necrotic and apoptotic cells in the cryptonin-treated MCF-7 cells (+/- Standard Error)**

| **Groups** | **Viable cell %** | **Necrotic cell %** | **Apoptotic cell %** |
|---|---|---|---|
| Control | 99.92±0.1 | 0±0 | 0.08±0.1 |
| 10 µg/ml | 98.89±5 | 4.48±4.9 | 0.63±0.3 |
| 25 µg/ml | 85.3±4.8 | 13.6±4.7 | 1.11±1 |
| 50 µg/ml | 1.07±1.1 | 96.52±1.5 | 2.41±1.6 |
| 100 µg/ml | 0.06±0.2 | 97.88±1.8 | 2.06±1.8 |
| 200 µg/ml | 2.14±1.8 | 97.86±1.8 | 0±0 |

**Table 2 Mean percentage values of the viable, necrotic and apoptotic cells in the cryptonin-treated MDA-MB 231 cells (+/- Standard Error)**

| **Groups** | **Viable cell %** | **Necrotic cell %** | **Apoptotic cell %** |
|---|---|---|---|
| Control | 97.63±1.4 | 0.34±0.9 | 2.43±1 |
| 10 µg/ml | 95.88±1.6 | 2.24±0.99 | 1.88±1. |
| 25 µg/ml | 95.53±2.9 | 2.19±2.7 | 2.26±1.7 |
| 50 µg/ml | 45.13±4.1 | 52.49±4.2 | 2.38±1.5 |
| 100 µg/ml | 0.04±0.1 | 98.09±1.5 | 1.87±1.5 |
| 200 µg/ml | 0±0 | 100±0 | 0±0 |

Figure 6: A) shows the dose-dependent number of ethidium bromide stained necrotic cell nuclei in MCF-7 cells. *Viable cell nucleus; necrotic cell nucleus; apoptotic cell nucleus. Bar: 100µm.* B) shows that cryptonin increases the number of necrotic cells in a dose-dependent manner, while reducing the number of viable cells in MCF-7 cells. *A significant difference was observed as compared to the control group (*p<0.05, **p<0.001; ***p<0.0001).*

### RESULTS OF SCANNING ELECTRON MICROSKOPE

### Effects of Peptide on MCF-7 Cell Membrane

Cryptonin administration has led to form pores and blebs on the cell membrane and caused the cell to shrink and contract.

### Effects of Peptide on MDA-MB 231 Cell Membrane

MDA-MB 231 cells, which were incubated in an antimicrobial peptide-free medium, were distinguished as cells with a rougher cell surface when compared to MCF-7 cells. In addition, few microvilli were distinguished on the cell surfaces. Cryptonin led to form pores and many small blebs. Also large protrusions were observed on the cell surface.

### Effects of Peptide on 3T3 Fibroblast Cell Membrane

3T3 fibroblast cells were observed as cells with very smooth cell surface. It has disrupted the cell membrane integrity by forming pores, slits and blebs in these cells treated with cryptonin.

### Measurement of Binding of the Peptide to Solid Phase Heparan Sulphate and Chondroitin Sulphate

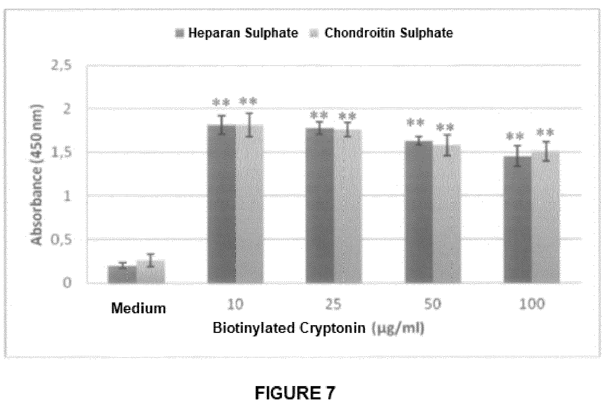

Figure 7 shows the binding of biotinylated cryptonin to heparan sulphate and chondroitin sulphate. *A significant increase was observed as compared to the medium (**p<0.001). Values of heparan sulphate are: Medium: 0.2±0.03, 10 µg*/*ml: 1.811±0.11, 25 µg*/*ml: 1.778±0.07, 50 µg*/*ml: 1.629±0.05, 100 µg*/*ml: 1.45±0.12. Values of chondroitin sulphate are: Medium: 0.26±0.07, 10 µg*/*ml: 1.811±0.13, 25 µg*/*ml: 1.754±0.08, 50 µg*/*ml: 1.58±0.12, 100 µg*/*ml: 1.506±0.11.*

Biotinylated cryptonin binds to heparan sulphate and chondroitin sulphate in a dose-dependent manner with a statistically significant increase as compared to the medium control.

### Measurement of Binding Affinity of the Peptide for MCF-7, MDA-MB 231 and 3T3 Fibroblast Cells

MCF-7 cells showed more affinity for a cryptonin concentration of 100 µg/ml as compared to a concentration of 200 µg/ml (^{a} p<0.0001). MDA-MB 231 cells exhibited the same affinity for cryptonin concentrations of 100 and 200 µg/ml (p>0,05). However, 3T3 fibroblast cells showed more affinity for a cryptonin concentration of 100 µg/ml (^{b} p<0.0001); 100 µg/ml of cryptonin showed less affinity for MDA-MB 231 cells as compared to MCF-7 cells (^{c} p<0.0001); the affinity for 3T3 cells is less than that of MCF-7 cells and MDA-MB 231 cells (^{d} p<0.0001; ^{e} p<0.0001). Similarly, the affinity of 200 µg/ml cryptonin for 3T3 cells is less than that of MCF-7 cells and MDA-MB 231 cells (^{f} p<0.0001; ^{a} p<0.0001).

The results obtained from the studies may be interpreted as follows;

Despite the great advances in the treatment of cancer, cancer is the leading cause of disease and death worldwide. Chemotherapy is a common treatment option for the treatment of advanced or metastatic disease. However, damage induced by chemotherapeutic drugs in healthy cells and tissues leads to undesirable side effects. On the other hand, cancer cells frequently exhibit resistance to chemotherapy by increasing the expression of drug detoxifying enzymes and carrier proteins that carry the drug out of the cell, altering interactions between drug and target, increasing DNA repair abilities, and defects in apoptosis pathways. Therefore, the discovery of agents that are not affected by the drug resistance mechanisms and that are not toxic to normal cells will be a significant advance in the treatment of cancer (Hoskin and Ramamoorthy, 2008; Wang et al., 2009).

Antimicrobial peptides encoded by the genes are ancestral effector molecules that have many functions in the natural immune system (Tossi et al., 2000; Ulm et al., 2012). These peptides constitute the first defense of the host organism exposed to bacteria and protect the natural flora. It is released in response to the presence of bacteria or expressed on epithelial surfaces for protection (Giangaspero et al., 2001). Until today, hundreds of different antimicrobial peptides have been characterized from numerous different organisms (http//: aps.unmc.edu/AP/main.php) and this number is increasing day by day. The diversity of antimicrobial peptides can reflect the adaptation of species to the microbial environment resulting from microorganisms in the environment and in food sources (Simmaco et al., 1998). Although these peptides differ in size, amino acid sequence and structure, they have two common and functionally important properties. These properties are that they have a net positive charge for interaction with anionic microbial surfaces and their ability to create amphipathic structure that allows them to be located in microbial membranes (Tossi et al., 2000). These two properties provide microbicidal activity via an interaction with microbial membranes, disruption of the membrane integrity, or binding to target molecules by passing through the membrane. In recent years, it has been reported that besides their microbicidal activity, some cationic antimicrobial peptides have killed mammalian cancer cells by inducing apoptosis or membrane permeabilization (Mader and Hoskin 2006; Suttmann et al., 2008; Cerön et al., 2010). However, many antimicrobial peptides have not been investigated for this activity. This study was carried out for revealing the effects of the antimicrobial peptide, the anticancer activity of which has not been investigated, on the breast cancer cell lines. It has been hypothesized that these peptides may have anticancer activity given their net positive charges and amphipathic characters for the selection of these peptides.

Neutrophils, eosinophils and macrophages, which constitute a significant part of the cell-dependent defense system in animals, produce polypeptides with a high cationic character. These peptides act by increasing bacterial membrane permeability or by inhibiting bacterial DNA synthesis, neutrophil-derived antimicrobial peptides such as a-defensins and LL-37 play an important role in innate immunity. In addition to killing infectious microorganisms, these peptides have many other functions, such as activation of the immune cells and chemotaxis (Bals and Hiemstra, 2004).

In this study, the anticancer activity of the cryptonin antimicrobial peptide α-helix structure defined in the hemolymph of cicada (Cryptotympana dubia) with a net charge of +8 was investigated for the first time. Said antimicrobial peptide reduced the cell viability in the MCF-7 and MDA-MB 231 cells in a dose-dependent manner. Cell viability-reducing effect of cryptonin was more in MCF-7 cells at doses of 7.5, 10, 25 and 50 µg/ml as compared to MDA-MB 231 cells. The fact that MCF-7 is more sensitive than MDA-MB 231 cells for cryptonin-induced cell death has been observed in the studies performed with temporin-lCEa peptide by Wang et al. (2012). This difference can be attributed to differences in membrane composition and fluidity of both cells and the easier interaction of the peptide with the MCF-7 cell membrane. However, cryptonin reduced the cell viability of 3T3 cells in a dose-dependent manner. Park et al. (2007) observed that cryptonin hemolyzes erythrocytes at a ratio of as low as 5% at a dose of 25-200 µg/ml. However, in the present study it was observed that fibroblast cell viability was significantly reduced at this dose range. Cryptonin has increased the LDH release and the number of ethidium bromide stained nuclei in a dose-dependent manner in both breast cancer cell lines. It has not led to any change in caspase-3 activity, and DNA fragmentation. In the light of these results, the impairment of porosity and membrane integrity in cells treated with cryptonin suggests that the cells are killed by the necrotic pathway.

Scanning electron microscopy was used to observe the morphological effect of cryptonin on breast cancer cell lines.

In the present study, it has been observed that the membrane integrity has been disrupted in MCF-7 and MDA-MB 231 cells incubated with the antimicrobial peptide, the anticancer activity of which has been investigated. The disruption in the membrane integrity is distinguished by the formation of pores and blebs of different sizes. Hoskin and Ramamoorthy (2008) suggested that the integration of the hydrophobic amino acids present in amphipathic antimicrobial peptides to a hydrophobic center, and formation a permanent amphipathic structure on the membrane may lead to porosity.

Cryptonin has exhibited reduced binding to heparan and chondroitin sulphate as the dose increases. In three different cells, cryptonin has showed reduced affinity in response to an increased dose without any statistical significance in MDA-MB 231 cells. This result can be due to the electrostatic repulsion force between the positively charged side chains because of being too cationic. Here again, if a rating is made in terms of binding to the peptide, the order is as follows: MCF-7, MDA-MB 231 and 3T3 fibroblast cells. This result is parallel to sensitiveness observed in the cell viability test for MCF-7 and MDA-MB 231 cells. Although 3T3 fibroblast cells has showed less binding to the peptide, its sensitiveness to the peptide is similar to that of MCF-7 according to the cell viability test results. The cytotoxic effect of cryptonin in fibroblast cells suggests that other factors such as cell membrane fluidity, transmembrane potential, secondary structure of the peptide, membran integrity disrupting mechanism of the peptide may also play a role.

## Claims

1. A peptide having SEQ ID NO: 1 for use in treatment or prophylaxis of cancer.

2. A peptide for use according to Claim 1, wherein said peptide is cryptonin.

3. A peptide for use according to Claim 1 or 2, wherein said cancer is selected from breast cancer, prostate cancer, skin cancer, gastric cancer, liver cancer, kidney cancer, lung cancer, colon cancer, ovarian cancer, testis cancer, bone cancer, ureter cancer, cervical cancer, endocrine gland cancers, vaginal cancer, endometrial cancer, duodenal cancer, head and neck cancer, Hodgkin's disease, vulva cancer and esophageal cancer.

4. A peptide for use according to Claim 3, wherein said cancer is breast cancer.

5. A peptide for use according to Claim 4, wherein said breast cancer is a disease selected from ductal carcinoma in situ and lobular carcinoma in situ, invasive ductal carcinoma and invasive lobular carcinoma, tubular carcinoma, invasive cribriform carcinoma, medullar carcinoma, mucinous carcinoma, neuroendocrine carcinoma, invasive papillary carcinoma, invasive micropapillary carcinoma, apocrine carcinoma, metaplastic carcinoma, lipid-rich carcinoma, secretory (juvenile) carcinoma, oncocytic carcinoma, and adenoid cystic carcinoma.

6. A peptide for use according to Claim 1 or 2, wherein said peptide has 24 amino acids and a linear structure.

7. A peptide for use according to Claim 2, wherein amino acids in the peptide are in D-form.

8. A peptide for use according to Claim 2, wherein said peptide has a net charge of +8.

9. A peptide for use according to Claim 4, wherein breast cancer is treated with necrotic cell death induced by cryptonin.

10. A peptide for use according to any of the preceding claims, wherein therapeutically appropriate dose range of said peptide is from 0.001 to 500 mg/kg/day.

11. A peptide for use according to any of the preceding claims, wherein the peptide has a net charge of +8 in order to disrupt the membrane integrity by interacting with the negatively charged molecules on the surface of the tumor cells.

12. A combination comprising a peptide having SEQ ID NO: 1, and at least one chemotherapeutic agent for use in treatment or prophylaxis of cancer.

13. A combination for use according to Claim 12, wherein said chemotherapeutic agent is selected from paclitaxel, docetaxel, vinorelbine, vincristine, vinblastine, doxorubicin, epirubicin, bleomycin, cisplatin, pentostatin, gemcitabine, mitomycin, etoposide, teniposide, topotecan, hydroxyurea, idarubicin, lomustine, thalidomide, 5-azacytidine, carboplatin, bortezomib, melphalan, 6-mercaptopurine, carmustine, oxaliplatin, daunorubicin, chlorambucil, irinotecan, semustine, cytarabine and busulfan.

14. A pharmaceutical composition comprising a peptide having SEQ ID NO: 1, and at least one pharmaceutically acceptable excipient for use in treatment or prophylaxis of cancer.

## Patentansprüche

1. Peptid mit der SEQ ID NO: 1 zur Verwendung bei der Behandlung oder Prophylaxe von Krebs.

2. Peptid zur Verwendung nach Anspruch 1, wobei das Peptid Cryptonin ist.

3. Peptid zur Verwendung nach Anspruch 1 oder 2, wobei der Krebs ausgewählt ist aus Brustkrebs, Prostatakrebs, Hautkrebs, Magenkrebs, Leberkrebs, Nierenkrebs, Lungenkrebs, Dickdarmkrebs, Eierstockkrebs, Hodenkrebs, Knochenkrebs, Harnleiterkrebs, Gebärmutterhalskrebs, Krebserkrankungen der endokrinen Drüsen, Vaginalkrebs, Endometriumkrebs, Zwölffingerdarmkrebs, Kopf-Halskrebs, Morbus Hodgkin, Vulvakrebs und Speiseröhrenkrebs.

4. Peptid zur Verwendung nach Anspruch 3, wobei der Krebs Brustkrebs ist.

5. Peptid zur Verwendung nach Anspruch 4, wobei der Brustkrebs eine Erkrankung ist, ausgewählt aus duktalem Karzinom in situ und lobulärem Karzinom in situ, invasivem duktalem Karzinom und invasivem lobulärem Karzinom, tubulärem Karzinom, invasivem cribriformem Karzinom, medullärem Karzinom, muzinösem Karzinom, neuroendokrinem Karzinom, invasivem papillärem Karzinom, invasivem mikropapillärem Karzinom, apokrinem Karzinom, metaplastischem Karzinom, lipidreichem Karzinom, sekretorischem (juvenilem) Karzinom, onkozytärem Karzinom und adenoidzystischem Karzinom.

6. Peptid zur Verwendung nach Anspruch 1 oder 2, wobei das Peptid 24 Aminosäuren und eine lineare Struktur aufweist.

7. Peptid zur Verwendung nach Anspruch 2, wobei die Aminosäuren in dem Peptid in D-Form vorliegen.

8. Peptid zur Verwendung nach Anspruch 2, wobei das Peptid eine Nettoladung von +8 aufweist.

9. Peptid zur Verwendung nach Anspruch 4, wobei Brustkrebs mit durch Cryptonin induziertem nekrotischem Zelltod, behandelt wird.

10. Peptid zur Verwendung nach einem der vorhergehenden Ansprüche, wobei der therapeutisch geeignete Dosisbereich des Peptids von 0,001 bis 500 mg/kg/Tag beträgt.

11. Peptid zur Verwendung nach einem der vorhergehenden Ansprüche, wobei das Peptid eine Nettoladung von +8 aufweist, um die Membranintegrität durch Wechselwirkung mit den negativ geladenen Molekülen auf der Oberfläche der Tumorzellen zu zerstören.

12. Kombination, umfassend ein Peptid mit der SEQ ID NO: 1 und mindestens ein Chemotherapeutikum zur Verwendung bei der Behandlung oder Prophylaxe von Krebs.

13. Kombination zur Verwendung nach Anspruch 12, wobei das Chemotherapeutikum ausgewählt ist aus Paclitaxel, Docetaxel, Vinorelbin, Vincristin, Vinblastin, Doxorubicin, Epirubicin, Bleomycin, Cisplatin, Pentostatin, Gemcitabin, Mitomycin, Etoposid, Teniposid, Topotecan, Hydroxyharnstoff, Idarubicin, Lomustin, Thalidomid, 5-Azacytidin, Carboplatin, Bortezomib, Melphalan, 6-Mercaptopurin, Carmustin, Oxaliplatin, Daunorubicin, Chlorambucil, Irinotecan, Semustin, Cytarabin und Busulfan.

14. Pharmazeutische Zusammensetzung, umfassend ein Peptid mit der SEQ ID NO: 1 und mindestens einen pharmazeutisch verträglichen Hilfsstoff zur Verwendung bei der Behandlung oder Prophylaxe von Krebs.

## Revendications

1. Peptide présentant la Séquence N° 1 pour utilisation dans le traitement ou la prophylaxie d'un cancer.

2. Peptide pour utilisation conforme à la revendication 1, lequel peptide est la cryptonine.

3. Peptide pour utilisation conforme à la revendication 1 ou 2, dans laquelle ledit cancer est choisi parmi les suivants : cancer du sein, cancer de la prostate, cancer de la peau, cancer de l'estomac, cancer du foie, cancer du rein, cancer du poumon, cancer du côlon, cancer des ovaires, cancer des testicules, cancer des os, cancer de l'uretère, cancer du col de l'utérus, cancers des glandes endocrines, cancer du vagin, cancer de l'endomètre, cancer du duodénum, cancer de la tête et du cou, maladie de Hodgkin, cancer de la vulve et cancer de l'œsophage.

4. Peptide pour utilisation conforme à la revendication 3, dans laquelle ledit cancer est un cancer du sein.

5. Peptide pour utilisation conforme à la revendication 4, dans laquelle ledit cancer du sein est une maladie choisie parmi les carcinome ductal in situ et carcinome lobulaire in situ, carcinome ductal invasif et carcinome lobulaire invasif, carcinome tubulaire, carcinome cribriforme invasif, carcinome médullaire, carcinome mucineux, carcinome neuroendocrinien, carcinome papillaire invasif, carcinome micro-papillaire invasif, carcinome apocrine, carcinome métaplasique, carcinome riche en lipides, carcinome sécrétoire (juvénile), carcinome oncocytaire et carcinome adénoïde kystique.

6. Peptide pour utilisation conforme à la revendication 1 ou 2, lequel peptide comporte 24 acides aminés et possède une structure linéaire.

7. Peptide pour utilisation conforme à la revendication 2, dans lequel les acides aminés du peptide se trouvent sous la forme D.

8. Peptide pour utilisation conforme à la revendication 2, lequel peptide présente une charge nette de +8.

9. Peptide pour utilisation conforme à la revendication 4, dans laquelle le cancer du sein est traité par mort de cellules par nécrose, induite par la cryptonine.

10. Peptide pour utilisation conforme à l'une des revendications précédentes, dans laquelle l'intervalle thérapeutiquement approprié de doses dudit peptide va de 0,001 à 500 mg/kg/jour.

11. Peptide pour utilisation conforme à l'une des revendications précédentes, lequel peptide présente une charge nette de +8 afin de détruire l'intégrité des membranes par interaction avec les molécules porteuses de charges négatives qui se trouvent à la surface des cellules tumorales.

12. Combinaison comprenant un peptide présentant la Séquence N° 1 et au moins un agent de chimiothérapie, pour utilisation dans le traitement ou la prophylaxie d'un cancer.

13. Combinaison pour utilisation conforme à la revendication 12, dans laquelle ledit agent de chimiothérapie est choisi parmi les paclitaxel, docétaxel, vinorelbine, vincristine, vinblastine, doxoru-bicine, épirubicine, bléomycine, cisplatine, pentostatine, gemcitabine, mitomycine, étoposide, téniposide, topotécan, hydroxyurée, idarubi-cine, lomustine, thalidomide, 5-aza-cytidine, carboplatine, bortézomib, melphalan, 6-mercapto-purine, carmustine, oxaliplatine, daunorubicine, chlorambucil, irinotécan, sémustine, cytarabine, et busulfan.

14. Composition pharmaceutique comprenant un peptide présentant la Séquence N° 1 et au moins un excipient pharmacologique-ment admissible, pour utilisation dans le traitement ou la prophylaxie d'un cancer.


### SEQUENCE LISTING

<110> ISTANBUL UNIVERSITESI
<120> Use of Cryptonin in Treatment of Breast Cancer
<130> 06-17716-A
<140> TR 2016/20086
   <141> 2016-12-29
<160> 1
<170> PatentIn version 3.5
<210> 1
   <211> 23
   <212> PRT
   <213> Cryptotympana dubia
<400> 1
   Gly Leu Leu Asn Gly Leu Ala Leu Arg Leu Gly Lys Arg Ala Leu Lys
   1               5                   10                  15
   Lys Ile Ile Lys Arg Leu Cys
               20